Europäisches Patentamt

European Patent Office

Office européen des brevets

(11) Publication number: **0 268 146 B1**

(12) **EUROPEAN PATENT SPECIFICATION**

(45) Date of publication of patent specification: **09.01.91**

(21) Application number: **87116236.8**

(22) Date of filing: **04.11.87**

(51) Int. Cl.⁵: **A 61 K 45/06,** A 61 K 31/505
// (A61K31/505,
31:415),(A61K31/505,
31:44),(A61K31/505, 31:50)

(54) Enhancement of prazosin.

(30) Priority: **05.11.86 US 927785**

(43) Date of publication of application:
**25.05.88 Bulletin 88/21**

(45) Publication of the grant of the patent:
**09.01.91 Bulletin 91/02**

(84) Designated Contracting States:
**AT BE CH DE ES FR GB GR IT LI LU NL SE**

(56) References cited:
**US-A-4 130 647**
**US-A-4 601 897**

(73) Proprietor: **MERRELL DOW
PHARMACEUTICALS INC.**
**P.O. Box 156300 2110 East Galbraith Road
Cincinnati Ohio 45215-6300 (US)**

(72) Inventor: **Dage, Richard C.**
**7825 Shadowhill Way
Cincinnati Ohio 45242 (US)**

(74) Representative: **Vossius & Partner
Siebertstrasse 4 P.O. Box 86 07 67
D-8000 München 86 (DE)**

Courier Press, Leamington Spa, England.

# EP 0 268 146 B1

**Description**

This invention relates to the synergistic enhancement of certain antihypertensives by the conjunctive use of certain cardiotonic agents. More specifically, this invention relates to the enhancement of the blood pressure lowering effect achieved with alpha$_1$-adrenoceptor antagonists by the conjunctive administration of cardiotonic agents possesssing the ability to specifically inhibit cyclic AMP-phosphodiesterase.

Still more specifically this invention relates to the synergistic enhancement of the antihypertensive effect of prazosin and prazosin-like compounds by the conjunctive administration of cardiotonic agents which are specific inhibitors of cardiac high affinity cyclic AMP-phosphodiesterase. These inhibitors are also known and identified as F-III PDE or Type 4 PDE inhibitors.

Prazosin, the hydrochloride salt of 1-(4-amino-6,7-dimethoxy-2-quinazolinyl)-4-(2-furoyl) piperazine, is the only commercially marketed compound of its type. Its exact mechanism of action is not precisely understood, but it is well known that it is unlike conventional alpha blockers in that it causes a decrease in total peripheral resistance related to the blockage of postsynaptic alpha$_1$-adrenoceptors. Other functionally equivalent agents, e.g., trimazosin, amiquinsin, leniquinsin, quinazosin appear to possess the same type of mechanism of action as does prazosin and, as functionally equivalent alpha$_1$-adrenoceptor antagonists, are included within the scope of this application.

The cardiotonic agents which synergistically enhance the antihypertensive effect of prazosin, and other alpha$_1$-adrenoceptor blocking agents, are those compounds which normally exert their positive ionotropic effects by selective inhibition of the specific molecular form of cardiac cyclic AMP-phosphodiesterase. Generally these are identified as cardiac cyclic AMP-phosphodiesterase high affinity cyclic AMP, F-III or Type 4 PDE inhibitors. Further, although there is no unaminity of thought as to the specific pharmacophore required for these selective inhibitors of cardiac cyclic AMP-phosphodiesterase, such compounds generally possess (1) the presence of a strong dipole (carbonyl) at one end of the molecule, (2) an adjacent acidic proton, (3) a methyl-sized lipophilic space, (4) a relatively flat overall topography and (5) a basic or hydrogen bond acceptor site opposite the dipole. These general characteristics are associated with those cardiotonic agents possessing the ability to selectively inhibit Type 4 PDE and which are generally associated with the pharmacophores possessed by such compounds as amrinone, enoximone, 4,5-dihydro-6-[4-(1H-imidazol-1-yl)phenyl]-5-methyl-(2H)-pyridazinone, piroximone, milrinone, isomazole, OPC 8212 (Otsuka), R-13-6438 (Roche), imazadan (Warner Lambert), ARL-57 (Tomae), Sulmazole, UD-CG-115 (Boeringer Ingelheim), UK—36,327 (Pfizer), UK—31,557, USV—2776 (U.S.V. Rohr), pimobendane and any other specific inhibitor of cyclic AMP-phosphodiesterase. This type of compound is the type of compound that selectively inhibits cyclic AMP-phosphodiesterase and as such is included within the scope of compounds which synergistically enhance the antihypertensive effect of alpha$_1$-adrenoceptor blocking agents such as prazosin and trimazosin.

In general, the specific cyclic AMP-phosphodiesterase inhibitors of this invention, (i.e., the above-described cardiotonic agents) when conjunctively administered with prazosin to effect their synergistic activity are administered at about the threshold dose to exert a blood pressure lowering effect, i.e., the minimum dose it takes to exert a statistically significant lowering of blood pressure. This threshold dose is readily determined by standard laboratory methodology. For example, assays such as the Spontaneously Hypertensive Rat and the Anesthesized Dog for testing blood pressure effects may readily be employed to determine the threshold dose for lowering blood pressure. The threshold dose may also be approximately calculated from an *in vitro* IC$_{50}$ value. For this purpose the *in vitro* IC$_{50}$ value is the micromolar quantity of compound needed to effect a 50% inhibition of the activity of cyclic AMP-phosphodiesterase on one (1) micromolar quantity of substrate. This IC$_{50}$ value, when multiplied by the appropriate fraction for intravenous and oral administrations will yield the dosage range, in mg per kg of body weight, necessary to effect its synergism with prazosin.

On the basis of spontaneously hypertensive rata data the multiplying factor is 1/10 to 1 for the intravenous dose and 1 to 10 for the oral dose. On the basis of the anesthesized dog data the multiplying factor is 1/100 to 1/3 for the intravenous dose and 1/3 to 2 for the oral dose. On the basis of projected human doses the multiplying factor is also 1/100 to 1/3 for intravenous dosing but 1/20 to 1/2 for oral dosing. For example, in the case of peroximone, the *in vitro* IC$_{50}$ value is 26 micromolar. Thus the dose range of peroximone to achieve synergism with prazosin in the SHR assay would be 2.6 to 26 mg/kg of body weight on intravenous administration and 26 to 260 mg/kg on oral administration.

Results demonstrating synergy are shown in Tables 1 and 2.

Analogous calculations may be made with data from dog studies and projected human doses. Table 1 shows the synergystic results of piroximone and prazosin. In the Table, line A shows the results of the control which is the vehicle administered at an oral dose of 5 ml/kg (using 28 animals). Line B shows the results of piroximone when administered at an oral dose of 25 mg/kg (using 12 animals). Line C shows the results of prazosin when administered at an oral dose of 0.1 mg/kg (using 24 animals). Line D shows the results of the combination of piroximone (25 mg/kg) and prazosin (0.1 mg/kg) administered orally (using 24 animals).

2

# EP 0 268 146 B1

## TABLE 1

### SYNERGISTIC EFFECT OF PIROXIMONE ON PRAZOSIN IN LOWERING BLOOD PRESSURE IN SPONTANEOUSLY HYPERTENSIVE RATS

| | CONTROL | SYSTOLIC BLOOD PRESSURE (mmHg, $\bar{x}\pm SE$) CHANGE FROM CONTROL AT | | | |
| --- | --- | --- | --- | --- | --- |
| | | 1 HOUR | 2 HOURS | 3 HOURS | 4 HOURS |
| A | 222 ± 5 | −15 + 5 | −9 ± 4 | −13 ± 5 | −13 ± 4 |
| B | 228 ± 5 | −23 ± 5 | −17 ± 6 | −18 ± 7 | −20 ± 5 |
| C | 223 ± 4 | −40 ± 4[+] | −40 ± 4[+] | −42 ± 4[+] | −35 ± 5[+] |
| D | 228 ± 4 | −81 ± 5[*+o] | −73 ± 4[*+o] | −68 ± 4[*+o] | −63 ± 3[*+o] |

\* Significant synergism, $p < 0.05$
\+ Significant effect compared to vehicle control, $p < 0.05$
° Significant difference from the effect of either agent alone

Table 2 shows the synergistic results of amrinone and prazosin. In the Table, line A shows the results of the control which is the vehicle administered at an oral dose of 5 ml/kg (using 12 animals). Line B shows the results of amrinone when administered at an oral dose of 55 mg/kg (using 12 animals). Line C shows the results of prazosin when administered at an oral dose of 0.1 mg/kg (using 12 animals). Line D shows the results of the combination of amrinone (55 mg/kg) and prazosin (0.1 mg/kg) administered orally (using 12 animals).

## TABLE 2

### SYNERGISTIC EFFECT OF AMRINONE ON PRAZOSIN IN LOWERING BLOOD PRESSURE IN SPONTANEOUSLY HYPERTENSIVE RATS

| | CONTROL | SYSTOLIC BLOOD PRESSURE (mmHg, $\bar{x}\pm SE$) CHANGE FROM CONTROL AT | | | |
| --- | --- | --- | --- | --- | --- |
| | | 1 HOUR | 2 HOURS | 3 HOURS | 4 HOURS |
| A | 220 ± 5 | −1 ± 8 | −4 ± 6 | −7 ± 7 | −19 ± 6 |
| B | 291 ± 7 | −26 ± 6[+] | −23 ± 5[+] | −21 ± 8 | −23 ± 7 |
| C | 220 ± 7 | −32 ± 3[+] | −31 ± 4[+] | −32 ± 4[+] | −31 ± 4 |
| D | 216 ± 7 | −82 ± 8[*+o] | −72 ± 8[*+o] | −70 ± 9[*+o] | −58 ±[*+o] |

\* Significant synergism, $p < 0.065$
\+ Significant effect compared to vehicle control, $p < 0.05$
° Significant difference from the effect of either agent alone, $p < 0.05$

It is preferable to administer the lowest amount of cyclic AMP-phosphodiesterase inhibitor in combination with the normal antihypertensive dose of prazosin. Futher enhancement of the antihypertensive effect of prazosin may be achieved by increasing the amount of the inhibitor of the cyclic AMP—PDE.

3

In the administration of the selective Type IV PDE inhibitors to enhance the antihypertensive effect of the alpha$_1$-adrenoceptor blocking agents, it is preferred to co-administer the two agents, but the enhancement will take place so long as it is administered to the patient while the alpha$_1$-adrenoceptor blocking agent is biologically available to effect its antihypertensive action. The synergistic effect will permit a lowering of the dose of the prazosin to avoid any untoward side effects and/or will permit a more effective blood pressure lowering effect, i.e., it will help stabilize the patient to normotensive conditions. Additionally, the combination will improve cardiac function in patients with congestive heart failure, particularly those suffering with hypertension.

**Claims for the Contracting States: AT BE CH DE FR GB IT LI LU NL SE**

1. Synergistically active composition for the treatment of hypertension comprising an alpha$_1$-adrenoceptor antagonist and an effectively enhancing amount of a cardiotonic agent capable of specifically inhibiting cardiac high affinity cyclic AMP-phosphodiesterase.
2. The composition according to claim 1 wherein the alpha$_1$-adrenoceptor antagonist is prazosin.
3. The composition of claim 2 wherein the cardiotonic agent is enoximone.
4. The composition of claim 2 wherein the cardiotonic agent is amrinone.
5. The composition of claim 2 wherein the cardiotonic agent is 4,5-dihydro-6-[4-(1H-imidazol-1-yl)phenyl]-5-methyl-(2H)-pyridazinone.
6. The composition of claim 2 wherein the cardiotonic agent is piroximone.
7. The composition of claim 2 wherein the cardiotonic agent is imadazan.
8. The composition of claim 2 wherein the cardiotonic agent is isomazole.
9. The use of a cardiotonic agent capable of specifically inhibiting cardiac high affinity cyclic AMP-phosphodiesterase for the production of a medicament for synergistically enhancing the effect of an α-adrenoceptor antagonist.

**Claims for the Contracting States: ES GR**

1. A method for preparing a synergistically active composition for the treatment of hypertension comprising combining an alpha$_1$-adrenoceptor antagonist and an effectively enhancing amount of a cardiotonic agent capable of specifically inhibiting cardiac high affinity cyclic AMP-phosphodiesterase.
2. The method according to claim 1 wherein the alpha$_1$-adrenoceptor antagonist is prazosin.
3. The method of claim 2 wherein the cardiotonic agent is enoximone.
4. The method of claim 2 wherein the cardiotonic agent is amrinone.
5. The method of claim 2 wherein the cardiotonic agent is 4,5-dihydro-6-[4-(1H-imidazol-1-yl)phenyl]-5-methyl-(2H)-pyridazinone.
6. The method of claim 2 wherein the cardiotonic agent is piroximone.
7. The method of claim 2 wherein the cardiotonic agent is imadazan.
8. The method of claim 2 wherein the cardiotonic agent is isomazole.

**Pantentansprüche für die Vertragsstaaten: AT BE CH DE FR GB IT LI LU NL SE**

1. Synergistisch wirksame Zusammensetzung für die Behandlung von Bluthochdruck, umfassend einen α$_1$-Adrenozeptor-Antagonisten und eine stärkend wirksame Menge eines kardiotonischen Wirkstoffs, der zur spezifischen Inhibierung der kardialen Hochaffinitäts-cyclischen AMP-Phosphodiesterase in der Lage ist.
2. Zusammensetzung nach Anspruch 1, wobei der α$_1$-Adrenozeptor-Antagonist Prazosin ist.
3. Zusammensetzung nach Anspruch 2, wobei der kardiotonische Wirkstoff Enoximon ist.
4. Zusammensetzung nach Anspruch 2, wobei der kardiotonische Wirkstoff Amrinon ist.
5. Zusammensetzung nach Anspruch 2, wobei der kardiotonische Wirkstoff 4,5-Dihydro-6-[4-(1H-imidazol-1-yl)-phenyl]-5-methyl-(2H) pyridazinon ist.
6. Zusammensetzung nach Anspruch 2, wobei der kardiotonische Wirkstoff Piroximon ist.
7. Zusammensetzung nach Anspruch 2, wobei der kardiotonische Wirkstoff Imazadan ist.
8. Zusammensetzung nach Anspruch 2, wobei der kardiotonische Wirkstoff Isomazol ist.
9. Verwendung eines kardiotonischen Wirkstoffs, der zur spezifischen Hemmung der kardialen Hochaffinitäts-cyclischen AMP-Phosodiesterase befähigt ist, zur Herstellung eines Arzneimittels zur synergistischen Potensierung der Wirkung eines α-Adrenozeptor-Antagonisten.

**Patentansprüche für die Vertragsstaaten: ES GR**

1. Verfahren zur Herstellung einer synergistisch wirksamen Zusammensetzung für die Behandlung von Bluthochdruck, umfassend eine α$_1$-Adrenozeptor-Antagonisten und eine stärkend wirksame Menge eines kardiotonischen Wirkstoffs, der zur spezifischen Hemmung der kardialen Hochaffinitäts-cyclischen AMP-Phosphodiesterase in der Lage ist.
2. Verfahren nach Anspruch 1, wobei der α$_1$-Adrenozeptor-Antagonist Prazosin ist.

3. Verfahren nach Anspruch 2, wobei der kardiotonische Wirkstoff Enoximon ist.

4. Verfahren nach Anspruch 2, wobei der kardiotonische Wirkstoff Amrinon ist.

5. Verfahren nach Anspruch 2, wobei der kardiotonische Wirkstoff 4,5-Dihydro-6-[4-(1H-imidazol-1-yl)-phenyl]-5-methyl-(2H) pyridazinon ist.

6. Verfahren nach Anspruch 2, wobei der kardiotonische Wirkstoff Piroximon ist.

7. Verfahren nach Anspruch 2, wobei der kardiotonische Wirkstoff Imazadan ist.

8. Verfahren nach Anspruch 2, wobei der kardiotonische Wirkstoff Isomazol ist.

**Revendications pour les Etats contractants: AT BE CH DE FR GB IT LI LU NL SE**

1. Composition à activité synergique pour le traitement de l'hypertension, comprenant un antagoniste des récepteurs $\alpha_1$-adrénergiques et une quantité potentialisante efficace d'un agent cardiotonique capable d'inhiber spécifiquement l'AMP cyclique-phosphodiésterase à forte affinité cardiaque.

2. Composition selon la revendication 1, dans laquelle l'antagoniste des récepteurs $\alpha_1$-adrénergiques est la prazosine.

3. Composition selon la revendication 2, dans laquelle l'agent cardiotonique est l'énoximone.

4. Composition selon la revendication 2, dans laquelle l'agent cardiotonique est l'amrinone.

5. Composition selon la revendication 2, dans laquelle l'agent cardiotonique est la 4,5-dihydro-6-[4-(1H-imidazole-1-yl)phényl]-5-méthyl-(2H)-pyridazinone.

6. Composition selon la revendication 2, dans laquelle l'agent cardiotonique est la piroximone.

7. Composition selon la revendication 2, dans laquelle l'agent cardiotonique est l'imazadan.

8. Composition selon la revendication 2, dans laquelle l'agent cardiotonique est l'isomazole.

9. Utilisation d'un agent cardiotonique capable d'inhiber spécifiquement l'AMP cyclique-phospho-diestérase à forte affinité cardiaque pour la production d'un médicament pour potentialiser l'effect d'un antagoniste des récepteurs $\alpha$-adrénergiques.

**Revendications pour les Etats contactants: ES GR**

1. Procédé pour préparer une composition à activité synergique pour le traitement de l'hypertension, comprenant l'association d'un antagoniste des récepteurs $\alpha_1$-adrénergiques et d'une quantité potentialisante efficade d'un agent cardiotonique capable d'inhiber spécifiquement l'AMP cyclique-phosphodiestérase à forte affinité cardiaque.

2. Procédé selon la revendication 1, dans lequel l'agent l'antagoniste des récepteurs $\alpha_1$-adrénergiques est la prazosine.

3. Procédé selon la revendication 2, dans lequel l'agent cardiotonique est l'énoximone.

4. Procédé selon la revendication 2, dans lequel l'agent cardiotonique est l'amrinone.

5. Procédé selon la revendication 2, dans lequel l'agent cardiotonique est la 4,5-dihydro-6-[4-(1H-imidazole-1-yl)phényl]-5-méthyl-(2H)-pyridazinone.

6. Procédé selon la revendication 2, dans lequel l'agent cardiotonique est la piroximone.

7. Procédé selon la revendication 2, dans lequel l'agent cardiotonique est l'imazadan.

8. Procédé selon la revendication 2, dans lequel l'agent cardiotonique est l'isomazole.